# EUROPEAN PATENT APPLICATION

(11) **EP 2 557 154 A2**
(43) Date of publication of application: **13.02.2013**
(21) Application number: 11766115.7
(22) Date of filing: 05.04.2011
(51) Int. Cl.: C12N 5/0775, C12N 5/02, A61K 35/12, A61P 9/00, A61P 37/00, A61P 19/00

(54) **METHOD FOR INCREASING ACTIVITY IN HUMAN STEM CELL**

(30) Priority: 05.04.2010 KR 20100031061; 07.06.2010 KR 20100053183
(71) Applicant: Seoul National University Hospital, Seoul 110-744 (KR)
(72) Inventor: KIM, Hyo-Soo, Seoul 137-040 (KR); KANG, Hyun-Jae, Seoul 110-054 (KR); LEE, Eun-Ju, Seoul 136-779 (KR)
(74) Representative: Goddar, Heinz J.
(86) International application number: PCT/KR2011/002358
(87) International publication number: WO 2011/126264

(57) **Abstract**

Provided are a method for preparing a highly active human mesenchymal stem cell, which includes forming a spherical cell aggregate by cultivating human mesenchymal stem cells against gravity; a highly active stem cell prepared thereby; a cell therapeutic agent including the stem cell aggregate; and a method for forming a spherical cell aggregate by cultivating human mesenchymal stem cells, wherein the amount of E-cadherin in the mesenchymal stem cell is increased during the cultivation.

## Description

### FIELD OF THE INVENTION

The present invention relates to a method for preparing a highly active human mesenchymal stem cell aggregate, a highly active stem cell aggregate obtained from the method, and a cell therapeutic agent containing the stem cell aggregate.

### BACKGROUND OF THE INVENTION

Stem cells are capable of differentiating into a variety of cells constituting tissues of an organism, and generally refer to undifferentiated cells obtainable from an embryo, a fetus and each tissue of an adult body. A stem cell differentiates into a specialized cell by a differentiation stimulus (environment); is capable of proliferation (expansion) by producing identical cells through cell division (self-renewal), unlike the differentiated cell whose cell division has been ceased; and is characterized by its plasticity of differentiation that it can differentiate into another cell under a different environment or by a differentiation stimulus.

Stem cells can be divided into, depending on their differentiation capacity, pluripotent, multipotent, and unipotent stem cells. Pluripotent stem cells are capable of differentiating into all cell types, *e.g*., embryonic stem cells (ES cells), and induced pluripotent stem cells (iPS). An example of multipotent and/or unipotent stem cells includes adult stem cells.

Embryonic stem cells are originated from an inner cell mass of blastocyte at the blastocyst stage. Such cells are characterized in that they can differentiate into cells of any type of tissues owing to their pluripotency of differentiating into any cell types, can be cultured in immortal and undifferentiated state, and can be inherited to the next generation, unlike adult stem cells through the preparation of germ cells (Thomson et al., Science, 282: 1145-1147 (1998); Reubinoff et al., Nat. Biotechnol., 18: 399-404 (2000)).

Human embryonic stem cells are prepared by isolating an inner cell mass only from a human blastocyst and culturing them. Currently, all the human embryonic stem cells prepared worldwide have been derived from frozen embryos remaining after a sterility treatment. Various approaches for using pluripotent human embryonic stem cells as a cell therapeutic agent have not yet completely successful due to the problems such as possibility of cancer development and immunological rejection.

As an alternative approach, induced pluripotent stem cells (iPS) have been brought to researchers' attention. Induced pluripotent stem cells are derived from differentiated adult cells via purposeful dedifferentiation into embryonic-type state using various methods. It has been reported that iPS cells have properties nearly identical to natural ES cells, in many aspects including gene expression, and differentiation capacity. In case of iPS cells, the possibility of immunological rejection can be avoided by using patient-derived cells as a source, but still have to confront the risk of cancer development.

Recently, mesenchymal stem cells have been suggested as an alternative for solving the risks associated with cancer development and immunological rejection. Mesenchymal stem cells are multipotent cells capable of differentiating into adipocytes, osteocytes, chondrocytes, myocytes, neurocytes, cardiomyocytes, hepatocytes, islet beta cells, angiocytes, etc. and have been reported to have an activity for regulating immune responses.

Mesenchymal stem cells can be separated from a variety of tissues, e.g., bone marrow, umbilical cord blood, adipose tissue and can be cultivated. However, their cell surface markers are different from each other according to their origins, and hence it is difficult to clearly define mesenchymal stem cells. In this regard, a mesenchymal stem cell is generally defined as its differentiation capability into osteoblasts, chondrocytes and myocytes, and characterized by its shape of whirlpool and its expression of standard cell surface markers, CD73(+), CD105(+), CD34(-), and CD45(-). In this connection, those mesenchymal stem cells having different genetic origins and/or background do not a show significant difference from one another based on the definition of the mesenchymal stem cells described above. However, they generally show significant differences *in vivo* activity. Also, in case mesenchymal stem cells are used as an allogeneic cellular therapeutic agent, available stem cell pool is limited. Hence, when selected mesenchymal stem cells show low *in vivo* activities, such cells, in some cases, cannot be replaced, and there are not many alternative choices.

In addition, in order to be used as a cell therapeutic agent, the mesenchymal stem cells generally have to meet a minimal cell number (about 1 x 10⁹ cells) required in the fields of cell therapy and/or regenerative medicine. The number of cells required to carry out the experiments becomes even greater when taking into consideration condition settings and establishing a standard. In case of conventional mesenchymal stem cells derived from various origins, at least 10 passages of *in vitro* experiment are required to obtain such amount of cells. In such case, the cells would become aged and modified, which would make them inadequate for use in the therapy.

Therefore, in order to efficiently use the mesenchymal stem cells as a cell therapeutic agent, it is required to develop a novel method which can maximize the therapeutic efficacy of the mesenchymal stem cells by inducing a high activity of the mesenchymal stem cell even with a small number thereof.

### SUMMARY OF THE INVENTION

It is an object of the present invention to provide a method of preparing a highly active stem cell aggregate from mesenchymal stem cells which are aged or have a relatively low *in vivo* activity.

It is another object of the present invention to provide a highly active mesenchymal stem cell aggregate prepared by the above method and a cell therapeutic agent comprising same.

In accordance with one aspect of the present invention, there is provided a method for preparing a highly active human mesenchymal stem cell aggregate, comprising culturing a human mesenchymal stem cell against gravity to form a spherical cell aggregate.

In accordance with another aspect of the present invention, there is provided a highly active mesenchymal stem cell aggregate prepared by the above method and a cell therapeutic agent comprising such stem cell aggregate.

In accordance with a further aspect of the present invention, there is provided a method for preparing a highly active human mesenchymal stem cell aggregate, comprising culturing a human mesenchymal stem cell to form a spherical cell aggregate, wherein the amount of E-cadherin in the mesenchymal stem cell is increased during the culture.

### BRIEF DESCRIPTION OF THE DRAWINGS

The above and other objects and features of the present invention will become apparent from the following description of the invention, when taken in conjunction with the accompanying drawings, which respectively show:
FIG. 1: the anchorage deprivation state of a human mesenchymal stem cell cultured in bFGF-free embryonic stem cell media.
FIG. 2: a spheroid formed by the anchorage deprivation of a human mesenchymal stem cell in a low attachment dish.
FIG. 3: a spheroid formed by the anchorage deprivation of a human mesenchymal stem cell cultured against gravity on a lid of culture dish.
FIG. 4: the results of left ventricular end-diastolic dimension (LVEDD) and left ventricular end-systolic dimension (LVESD) showing the evaluated outcome of ischemic heart disease.
FIG. 5: the results of left ventricular end-ejection fraction (LVEF) and left ventricular end-fractional shortening (LVFS) showing the evaluated outcome of ischemic heart disease.
FIG. 6: the results of the infarcted wall thickness and infarcted area showing the evaluated outcome of ischemic heart disease.
FIG. 7: the significantly higher number of cells remaining near the ischemic heart in a Spheroid group (injected with spherical cell aggregates) in comparison with a Naïve group (injected with non-spheroid forming cells).
FIG. 8: the expressions of sacomeric actinin (FIG. 8a) and connexin 43 (FIG. 8b) observed in a spheroid group.
FIG. 9: the expression of isolectin B4 as a vessel-specific marker and the quantified levels thereof, to observe effects on angiogenesis.
FIG. 10: the expression of isolectin B4, to observe whether injected mesenchymal stem cells have differentiated into angiocytes.
FIG. 11: the result showing no formation of spheroid when EDTA was added.
FIG. 12: the results of a Western blot analysis for the detection of Ca²⁺-dependent cell adhesion molecules, *i.e*., N-cadherin and E-cadherin, during spheroid formation.
FIG. 13: the result of a spheroid formation of mesenchymal stem cells when the function of E-cadherin is inhibited.
FIG. 14: the effect on a spheroid formation when E-cadherin is overexpressed by using E-cadherin adenoviral vector.
FIG. 15: the result of activities of an extracellular signal-regulated kinase (ERK) and V-akt murine thymoma viral oncogene homolog (AKT) in accordance with the spheroid formation.
FIG. 16: the effect of E-cadherin on activities of ERK and AKT.
FIG. 17: the result of activities of ERK and AKT when E-cadherin is overexpressed by using E-cadherin adenoviral vector.
FIG. 18: the effect of E-cadherin on the growth of mesenchymal stem cells.
FIG. 19: the effect of E-cadherin on cell death of mesenchymal stem cells.
FIG. 20: the effect of E-cadherin on the release of vascular endothelial growth factor (VEGF) of mesenchymal stem cells.
FIG. 21: the result of mixed lymphocyte reaction (MLR) to determine the degree of *ex vivo* immune cell responses using two types of umbilical cord blood derived mesenchymal stem cells (UCB-MSCs) originated from different origins.
FIG. 22: the ELISA analysis result of supernatants obtained from each MLR cell culture to determine FGE₂ concentration.
FIG. 23: the effect of spheroid formation on immune system observed by using an immunocyte marker, F4/80.
FIGs. 24A and 24B: the chondrocyte (live/dead) staining assay results to determine the inhibitory effect on cell death induced by spheroid formation of mesenchymal stem cells; and the graph of cell viability calculated therefrom.
FIG. 25: the results of naked eye analysis and tissue staining analysis in damaged cartilage site of defective cartilage rabbit model (after 10 weeks) to determine the regeneration effect on chondrocytes induced by spheroid formation of mesenchymal stem cells.
FIG. 26: the expression results of a differentiation activator during the induction of differentiation into pneumocytes compared between the spheroids prepared by hanging drop and bioreactor methods.

### DETAINED DESCRIPTION OF THE INVENTION

The present invention provides a method of preparing a highly active human mesenchymal stem cell aggregate. Specifically, the present invention provides a method of preparing a highly active human mesenchymal stem cell aggregate, comprising culturing human mesenchymal stem cells against gravity to form a spherical cell aggregate.

As used herein, the terms "stem cell aggregate", "aggregate", or "spheroid", used interchangeably, refer to a spherical stem cell aggregation formed by culturing stem cells.

The human mesenchymal stem cell used in the present invention has no limitations on the genetic background and/or the origin thereof. For example, such human Mesenchymal stem cell may include umbilical cord blood derived mesenchymal stem cells (UCB-MSCs), adipose-derived mesenchymal stem cells (AD-MSCs), bone marrow-derived mesenchymal stem cells (BM-MSCs), and the like, preferably, UCB-MSCs.

In the present invention, the culture of the human mesenchymal stem cells to form the spherical cell aggregate may be carried out in a culture drop positioned against gravity. In this regard, the spherical cell aggregate may be formed from 300 to 30,000 cells per drop, preferably 1,000 to 30,000 cells per drop, so as to obtain a spherical cell aggregate having a high therapeutic efficacy.

The culturing method of the stem cells against gravity results in a great number of stem cell aggreates having a uniform size, which enhance the therapeutic effectiveness.

In the present method, the culture medium may be a serum-free medium containing a serum replacement (SR). Any commercially available SR may be used in the present invention, and the SR concentration in the medium may be adjustable, if necessary, preferably 20% (v/v).

The serum-free medium may be a human embryonic stem cell culture medium that does not contain serum and basic fibroblast growth factor (bFGF).

The present invention also provides a method for preparing a spherical cell aggregate, comprising culturing human mesenchymal stem cells, wherein amount of E-cadherin in the mesenchymal stem cells is increased during the culture.

The amount of E-cadherin in the mesenchymal stem cells may be increased by introducing an E-cadherin expression vector into the mesenchymal stem cells. For example, the expression vector may be an adenovirus vector comprising an E-cadherin gene.

Further, the culture of the mesenchymal stem cells to form the spherical cell aggregate may be carried out by culturing the stem cells against gravity employing a culture medium mentioned above, or by anchorage deprivation employing a low-attachment culture dish. In case of the anchorage deprivation, it may further contain a step of separating produced spherical cell aggregates from other cells not included in the spherical cell aggregates. In such separation step, any tool for separating the spherical cell aggregates from single cells by size preferably, a strainer may be employed.

Also, the spherical cell aggregate may be formed by culturing the mesenchymal stem cells in the culture medium mentioned above by employing a three-dimensional bioreactor (or a spinner); culturing the mesenchymal stem cells in a conventional attachment container with stirring to reduce the opportunity of the stem cells to attach on the bottom of the container; culturing single cells under a stress-inducing condition, *e.g*., hypoxia or a low temperature below a room temperature. It may be also possible to form the spherical cell aggregate by culturing a particular number of the stem cells in a plate such as AggreWell^{™} having a micro-well structure on the bottom, or putting single cells in a non-attachment container or an injector of stem cell therapeutic agent.

Further, the present invention provides a highly active human mesenchymal stem cell aggregate prepared by the above method.

The stem cell aggregate according to the present invention exhibits good *in vivo* tissue regeneration and treatment efficacy, high *in vivo* viability, and good differentiation efficiency into tissue cells.

Furthermore, the present invention provides a cell therapeutic agent comprising the highly active human mesenchymal stem cell aggregate.

The cell therapeutic agent according to the present invention may be used for generating adipocytes, osteocytes, chondrocytes, myocytes, neurocytes, cardiomyocytes, hepatocytes, islet beta cells, angiocytes, or pneumocytes.

Further, the cell therapeutic agent according to the present invention may be used for any one selected from the group consisting of treatment of a pulmonary disease; suppression or treatment of an inflammation caused by a pulmonary disease; regeneration of a pulmonary tissue; and suppression of fibrosis in a pulmonary tissue. Preferably, it may suppress or relieve inflammation due to a pulmonary disease, and fibrosis.

The cell therapeutic agent according to the present invention may be used for the treatment of a cardiovascular disease or chondrogenesis.

Moreover, the cell therapeutic agent according to the present invention may increase immunomodulatory functions and reduce any one selected from the immunifacient activity, penetration of immunocytes and immunogenicity. It may also suppress an inflammatory reaction.

Further, the present invention provides a method for mass producing highly active human mesenchymal stem cells on a large scale using a bioreactor.

A bioreactor is a system or device that maintains and supports a biologically active environment. The human mesenchymal stem cells may induce to form the spherical cell aggregates in the bioreactor, the highly active human mesenchymal stem cell aggregates without capable of growing contact inhibition can be produced on a large scale, by continuously culturing the spherical cell aggregates thus formed in the bioreactor. In other words, the mesenchymal stem cells may form the spherical cell aggregates in said culture medium mentioned above in the bioreactor by using centrifugal force generated by stirring, and the spherical cell aggregates thus obtained may be further cultured in the same culture medium to yield highly active human spherical mesenchymal stem cells on a large scale.

The present method may enhance the activity of mesenchymal stem cells which are aged or have a relatively low *in vivo* activity, which maximizes practicality and treatment efficacy of the mesenchymal stem cells as a cell therapeutic agent. Also, it may be used as a standardized method applicable to all of the mesenchymal stem cells having different genetic background and/or the origin and can be very useful for development and selection of allogeneic cell therapeutic agents.

In addition, the present invention can maximize effectiveness of human mesenchymal stem cells which allows researchers to come up with a proper number of high functional human mesenchymal stem cells required in the fields of cell therapy and regenerative medicine. Also, the present invention enables a mass production of the highly active human mesenchymal stem cells.

Ultimately, the present invention may augment the efficiency of human mesenchymal stem cells which could promote practical use of a cell therapeutic agent, and eventually contribute to the development of a therapeutic drug for treating cardiovascular diseases, nervous system disorders, etc.

The present invention is further disclosed in the following Examples. It should be understood that these Examples, while indicating preferred embodiments of the invention, are given by way of illustration only and are not intended to limit the scope of the invention.

### Examples

In the present invention, human umbilical cord blood mesenchymal stem cells purchased from MEDIPOST Co., Ltd. (Korea) were used. The stem cells were identified and classified as "human umbilical cord blood derived mesenchymal stem cells (UCB-MSCs)" after the identification test of human mesenchymal stem cells, and the test includes the expressions of positive cell markers (*i.e*., CD29, CD44, CD73, CD105, CD166, and HLA-ABC) of at least 95% and negative cell markers (*i.e*., CD34, CD45, and HLA-DR) of less than 5%, and the confirmation of multipotency of the mesenchymal stem cells.

### Example 1: Inducting spheroid formation of human mesenchymal stem cells

### (1) Culture medium for inducting spheroid formation

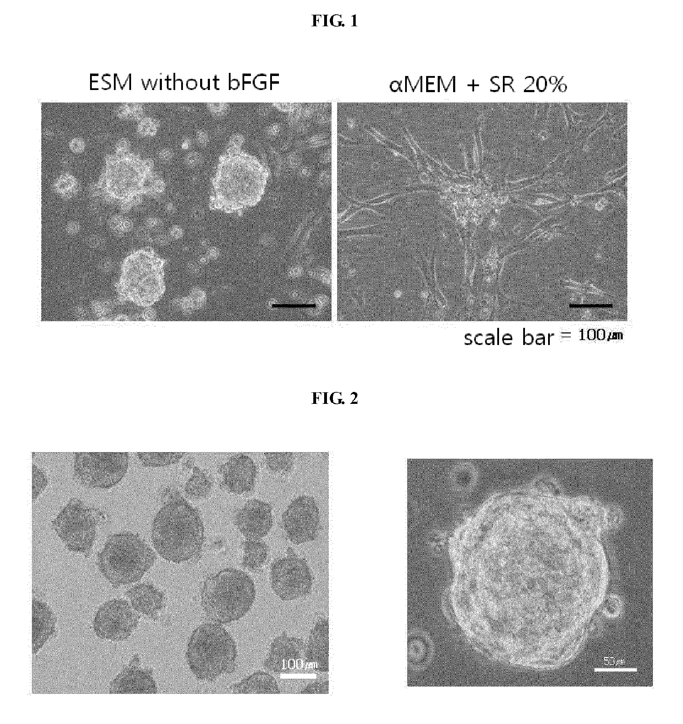

The above mesenchymal stem cells were cultured in a conventional culture medium for mesenchymal stem cells, α-MEM medium (Invitrogen) supplemented with a serum replacement (SR) by employing a low attachment dish to allow anchorage deprivation. However, it was unsuccessful to induce anchorage deprivation (*see* FIG. 1A).

Next, the mesenchymal stem cells were cultured in an embryonic stem cell media (ESM) from which basic fibroblast growth factor (bFGF) was removed (hereinafter, bFGF-free ESM) by employing a low attachment dish to allow the anchorage deprivation. It was successful to induce the anchorage deprivation (*see* FIG. 1B). The culture medium did not contain any fetal bovine serum (FBS), but contained DMEM/F-12 (Invitrogen), 20% Knock out SR (Invitrogen), 0.1 mmol/L β-mercaptoethanol (Sigma), 1% non-essential amino acids (Invitrogen), 50 IU/mL penicillin and 50 mg/mL streptomycin (Invitrogen).

### (2) Method of spheroid formation

The present inventors utilized two different methods for spheroid formation of the human mesenchymal stem cells. Successful formation of spheroid was accomplished by both of the following methods.

First, the human mesenchymal stem cells were cultured in the bFGF-free ESM prepared in step (1) by employing a low attachment dish to induce the spheroid formation, and the result is shown in FIG. 2. Spherical cell aggregates were prepared and they were then separated from other non-spheroid forming cells by using a strainer.

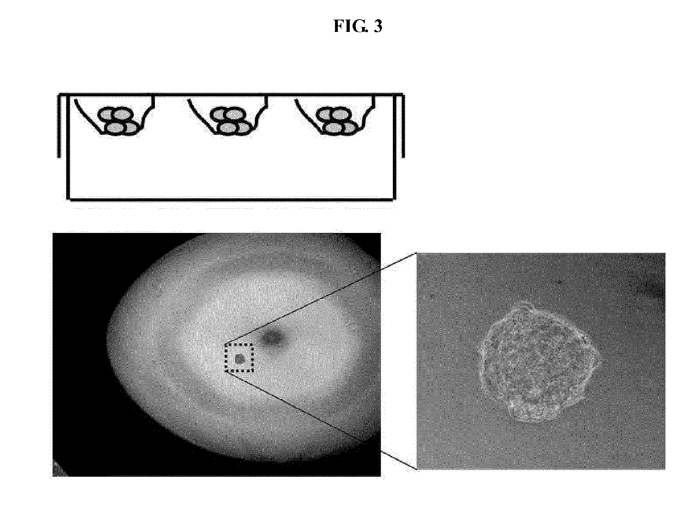

As another method, the mesenchymal stem cells in the same culture medium were inoculated on a lid of culture dish at a concentration of 300 to 3,000 cells/20 µl of culture medium. Then, the lid was turned upside down and cultured against gravity to induce spheroid formation. The result is shown in FIG. 3. This method has advantages in that the number of the cells can be controlled and the formed spheroid may have a uniform size, which contribute to stem cell aggregates with a high therapeutic effectiveness. Therefore, the stem cell aggregates used in the present invention were prepared by this method, unless otherwise mentioned.

### Example 2: Effect by spheroid formation - in vivo activity

*In vivo* activity of mesenchymal stem cells was evaluated using a rat model having ischemic heart disease. The rat model of ischemic heart disease was prepared by coronary artery ligation to induce ischemia.

The rat model of ischemic heart disease was divided into three groups for the evaluation: A group (Spheroid) injected with the spherical cell aggregates prepared in (2) of Example 1; a group (Dissociate) injected with single cells prepared from the spherical cell aggregates; and a group (Naïve) injected with cells which were not induced to form any spherical cell aggregates. At least 7 rats were used for each group.

### (1) Electrocardiogram measurement

Baseline electrocardiogram was measured 4 days after the rat model was prepared, and the stem cells were injected into the rats 7 days after the rat model was prepared. Specifically, the stem cells or cell aggregates were injected into a site near myocardium where ischemic heart disease was induced in the rat model using a Hamilton syringe made with frictionless glass. The number of the mesenchymal stem cells injected was adjusted to 1 x 10⁵ cells per rat. Electrocardiogram measurements were taken 4 and 8 weeks after the injection. The results of left ventricular end-diastolic dimension (LVEDD), left ventricular end-systolic dimension (LVESD), left ventricular end-fractional shortening (LVFS), and left ventricular end-ejection fraction (LVEF) were diagramed and improvement of the disease was evaluated. LVFS is defined as LVEDD-LVESD/LVEDD, and LVEF is defined as LVEDD²_LVESD²/LVEDD². The lower values of LVEDD and LVESD, and the higher values of LVFS and LVEF represent better improvement of the ischemic heart disease.

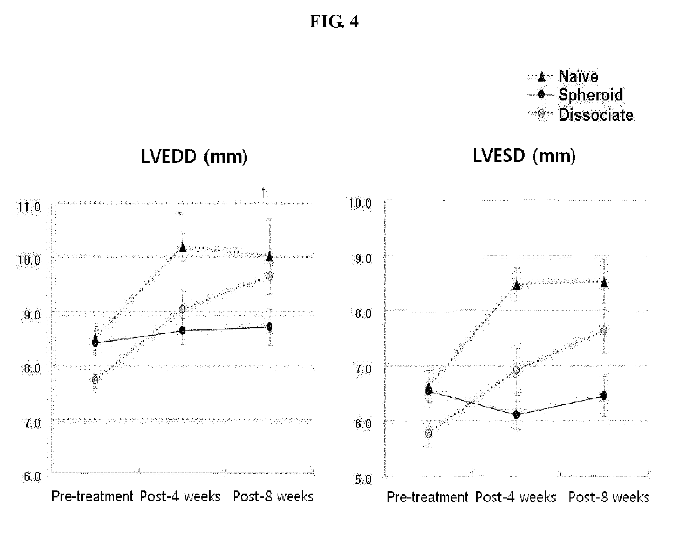

As shown in FIG. 4, Spheroid and Dissociate groups resulted in lower LVEDD and LVESD values as compared with those of Naive group. Particularly, Spheroid group resulted in significantly lower LVEDD and LVESD values when compared with those of Naïve group. The values of Spheroid group were still lower than those of Dissociate group.

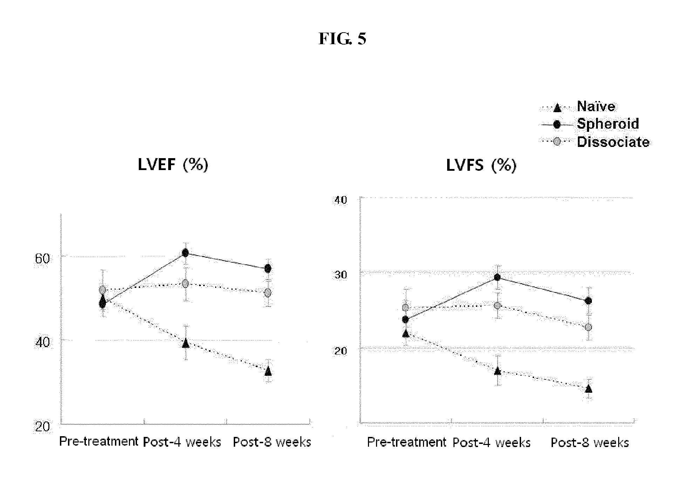

Also, as shown in FIG. 5, Spheroid and Dissociate groups resulted in higher LVFS and LVEF values as compared with those of Naïve group. Particularly, Spheroid group resulted in significantly higher LVFS and LVEF values when compared with those of Naïve group. The values of Spheroid group were still higher than those of Dissociate group.

From the above results, the injection of the spherical cell aggregates exhibited a high improvement of the diseases.

### (2) Comparison of heart size and fibrosis

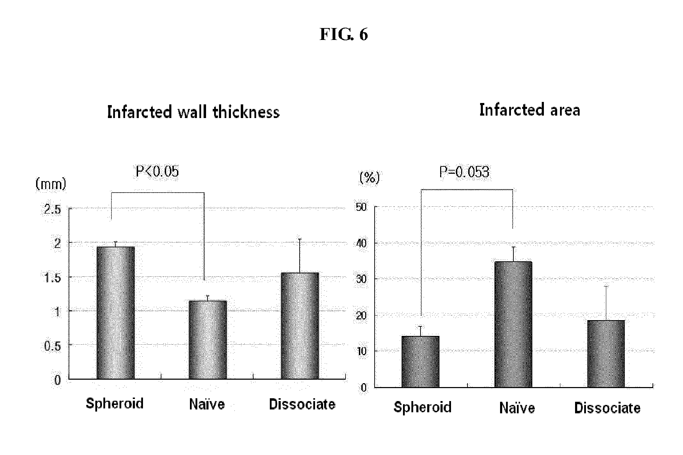

Besides the electrocardiogram measurements, the effects on overall thickness of heart wall and fibrosis by the injection of the spherical cell aggregates were investigated. FIG. 6 shows damaged results of the infarcted wall thickness and infarcted area.

Generally, when ischemia occurs in a heart, the thickness of the heart wall decreases owing to fibrosis of the heart wall, and loss of mobility and volume expansion follows. As shown in FIG. 6, Spheroid group significantly reduced the conventional symptoms of ischemia, *i.e*., thinning of the heart wall and progress of fibrosis, as compared with Naïve group. Also, Spheroid group reduced the symptoms of thinning of the heart wall and progress of fibrosis compared with Dissociate group.

### (3) Histological analysis of heart

To find out causes with regard to the results of the *in vivo* activity (improvement) in the rat model of ischemic heart disease, the heart was histologically analyzed. In order to easily track the mesenchymal stem cells remaining after the injection, the stem cells were stained with DiI and then injected into the rat models.

DiI (1,1'-dioctadecyl-3,3,3',3-tetramethylindocarbocyanine perchlorate) is a hydrophobic and lipophilic dye which stains the entire cell in red by binding to bilayer lipid membrane of the cell. In order to observe the injected cells in an organ, a sample of heart tissue was taken, dyed with DAPI, and then observed using a fluorescence microscope. DAPI (4',6-diamidino-2-phenylindole) is a blue fluorescent material that binds strongly to the minor groove of A-T cluster in a double-helix DNA.

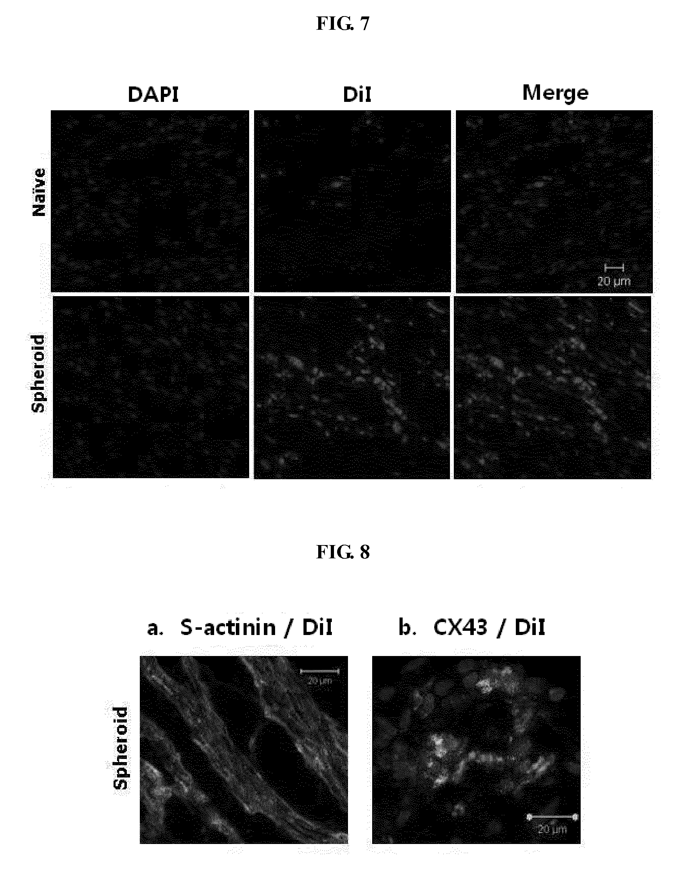

As shown in FIG. 7, it was observed that the mesenchymal stem cells remained near the ischemic heart in Spheroid group were noticeably greater than Naive group. In other words, a great number of DiI-dyed cells (red) indicate that the spherical cell aggregate exhibited an excellent effect on the survival rate of the injected cells.

Also, in order to investigate whether the mesenchymal stem cells remaining near the ischemic heart were differentiated into cardiomyocytes lost by ischemia, the expression of sarcomeric actinin (S-actinin), as a cardiomyocyte-specific marker, was observed. Additionally, the expression of connexin 43 (CX43), which plays an important role in connection with the remaining cardiomyocytes, was also observed.

As shown in FIG. 8, the expression of S-actinin was observed (parts dyed in green) in Spheroid group (*see* FIG. 8a). This suggests that the mesenchymal stem cells remaining near the ischemic heart were differentiated into cardiomyocytes lost by ischemia. Also, the expression of connexin 43 which plays an important role in cardiomyocyte function was observed (parts dyed in green) in Spheroid group (*see* FIG. 8b).

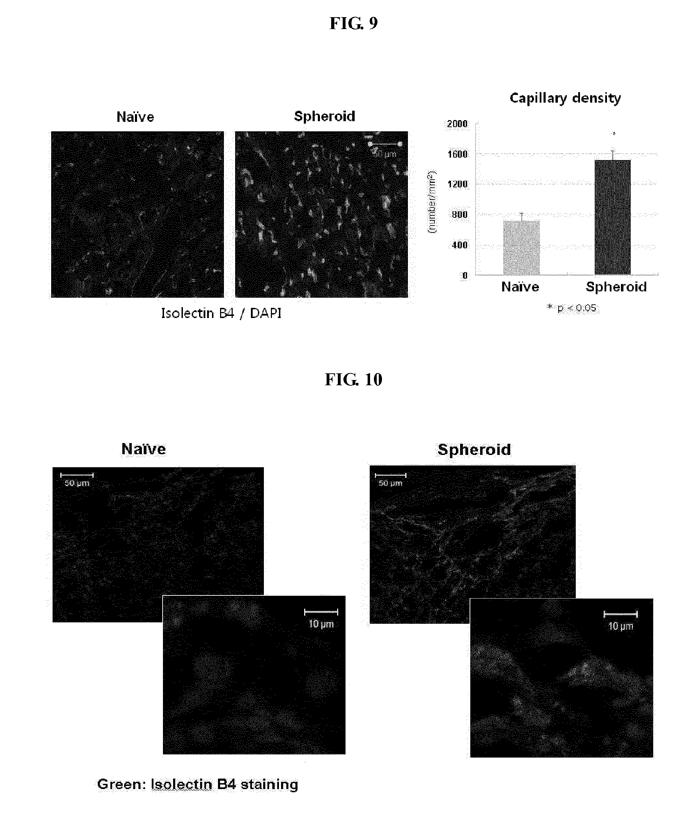

In addition, the effect on angiogenesis, the most important factor in improving the ischemic heart disease, was investigated. Specifically, the expression of isolectin B4, as a vessel-specific marker, was observed, and the result was quantified and shown in FIG. 9.

As shown in FIG. 9, Spheroid group injected with spherical cell aggregates resulted in remarkable expression of isolectin B4 (parts dyed in green) in comparison to that of Naïve group. This indicates Spheroid group was over two-fold angiogenic than Naïve group as shown in quantified diagram (cell number per mm²).

Also, a test was conducted to confirm whether the angiogenesis was induced by angiocytes differentiated from the mesenchymal stem cells remaining near the ischemic heart. Specifically, the cells were stained with DiI and isolectin B4, and the result was analyzed.

As shown in FIG. 10, Spheroid group injected with the spherical cell aggregates resulted in remarkable expression of isolectin B4 (parts dyed in green) in comparison to that of Naïve group. This indicates the spheroid formation contributes to the differentiation of the mesenchymal stem cells into angiocytes.

From the above results, the injection of the spherical stem cell aggregates prepared by culturing the human mesenchymal stem cells by anchorage deprivation into the rat model of the ischemic heart disease, exhibited an improved treatment effect compared to cells that did not from spheroid or single cells re-separated from the spherical cell aggregates. Moreover, it was confirmed that the improved treatment efficacy of the ischemic heart disease shown in Spheroid group resulted from the significantly improved survival rate of the mesenchymal stem cells, and differentiation efficacy into cardiomyocytes and angiogenesis, induced by the spheroid formation of the mesenchymal stem cells.

In conclusion, the induction of spheroid formation in mesenchymal stem cells activates the mesenchymal stem cells. Furthermore, it is clear that the activities of the mesenchymal stem cells were increased by maintaining the spheroid formed rather than utilizing single cells reseparated from the spherical cell aggregates.

### Example 3: Analysis of spheroid formation mechanism (in vivo activity)

A test was conducted to analyze the mechanism of spheroid formation that caused different *in vivo* activity as disclosed in Example 2.

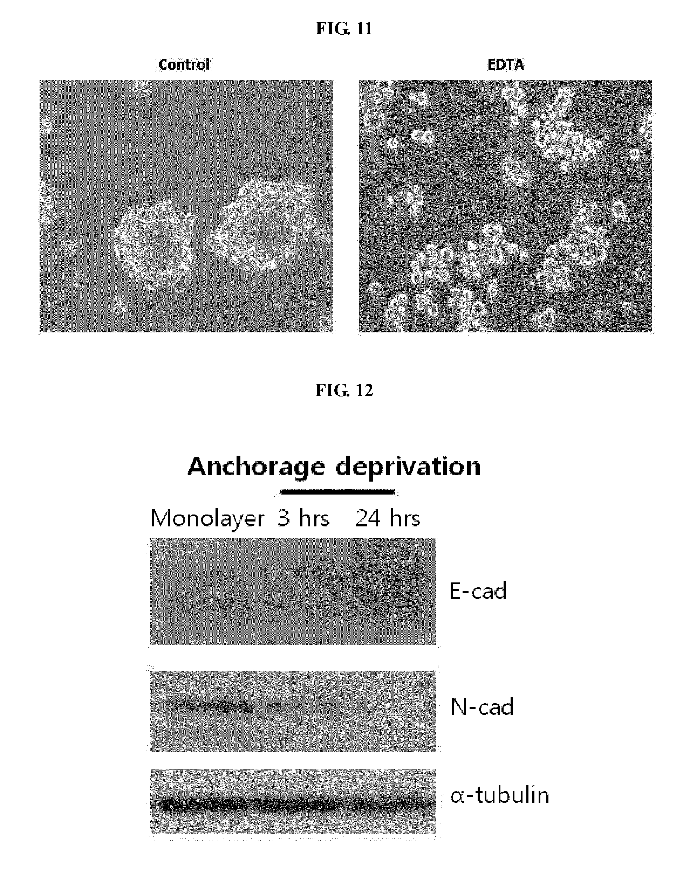

First, EDTA was added to the stem cells induced to the spheroid formation in bFGF-free ESM by employing a low attachment dish, to chelate calcium ion (Ca²⁺) which plays an important role as a cell adhesion factor. As shown in FIG. 11, the spheroids were not formed in the presence of EDTA. In other words, the spheroid formation of the mesenchymal stem cells was interrupted by the addition of EDTA as a Ca²⁺ chelator, and thus, the spheroid formation is considered to be carried out by Ca²⁺-dependent cell adhesion molecule(s).

Hence, the expressions of two different Ca⁺²-dependent cell adhesion molecules, *i.e*., N-cadherin and E-cadherin during the spheroid formation were examined by Western blot analysis. As shown in FIG. 12, the expression of N-cadherin (abcam, ab18203) diminished when the spheroid formation was induced by the anchorage deprivation, while the expression of E-cadherin (abcam, ab1416), a counterpart of N-cadherin, increased when the spheroid formation was induced. α-tubulin was used as a control.

In Western blot analysis, the stem cells were dissolved in a reducing agent (Lysis PreMix (4°C stock) + NaF (10 M, x100) + orthovanadate (200 mM, x200) + protease inhibitor cocktail (1 tablet/10 mL)), subjected to SDS-polyacrylamide gel electrophoresis, transferred to PVDF transfer membrane (Millipore) and subjected to primary antigen-antibody reaction and secondary antigen-antibody reaction using anti-rabbit IgG and anti-mouse IgG to investigate protein expression.

As a result, it was confirmed that E-cadherin may play as a key factor for spheroid formation of the human mesenchymal stem cells. Therefore, the present inventors conducted a further following test to investigate the effects of E-cadherin on the spheroid formation and spheroid activity of the mesenchymal stem cells.

### Example 4: E-cadherin activity - Effects on spheroid formation and high activity of spherical cell aggregates

### (1) Effects on spheroid formation

First, the effect of the mesenchymal stem cell on the spheroid formation when the function ofE-cadherin was blocked was investigated.

Specifically, intercellular adhesion function of E-cadherin was eliminated by using an antibody (Clone, DECMA-1) which is known to neutralize E-cadherin by recognizing the cell membrane sites of E-cadherin.

This process was accomplished by adding E-cadherin neutralization antibody in an amount of 2~10 µg/mL or IgG when inducing spheroid formation of the stem cells in bFGF-free ESM by employing the low attachment dish.

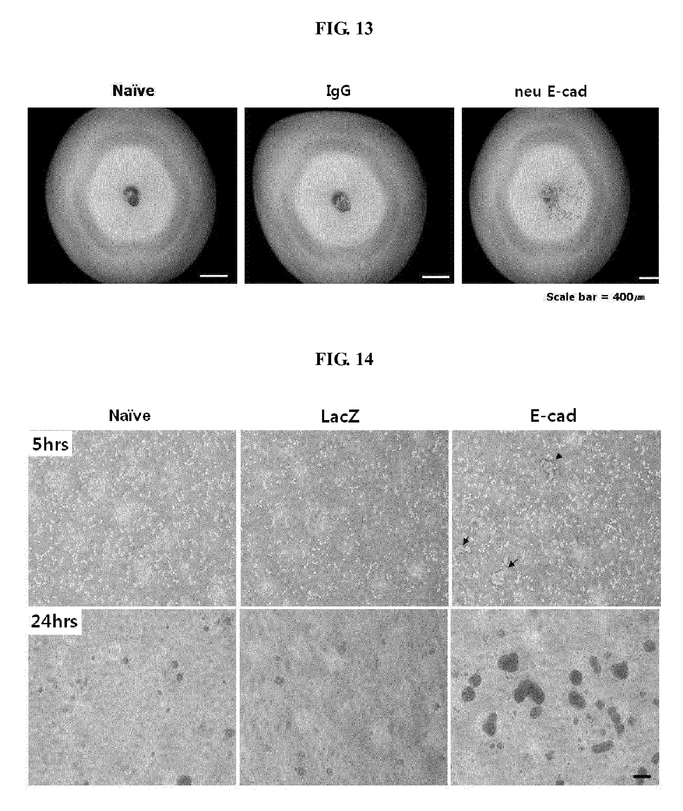

As shown in FIG. 13, IgG-treated (IgG) group and untreated control (Naïve) group allowed the spheroid formation, whereas inhibited E-cadherin function group (Neu E-cad) did not allow spheroid formation. Naïve group was used as a control to verify that the antibody treated group was not carried out in a special condition such as apoptosis or activation, and hence had the same result as compared to IgG-treated group.

Additionally, the effect of E-cadherin on spheroid formation was reevaluated using E-cadherin overexpressing adenoviral vector. The same vector as E-cadherin overexpressing adenoviral vector except comprising LacZ gene instead of E-cadherin was used as a control group.

CMV promoter was used, and adenoviral vector was quantified after induction of viral packaging at 293 cells. In the same manner of the conventional viral vector transduction, a viral supernatant was added to the mesenchymal stem cells having 70% confluence by adherent culture to induce the expression of E-cadherin. After 24 hours for transduction, the cells were allowed to stabilize for 24 hours and separated to single cells. The separated single cells were induced to the spheroid formation in bFGF-free ESM by employing the low attachment dish, and the samples were collected.

Adenoviral vector-untreated group (Naïve group) was used as a control group to compare with the adenoviral vector-treated groups (E-cadherin or LacZ group). The adenoviral vector was treated to the cells for 4 hours to induce the spheroid formation, respectively, and their effectiveness for induction of spheroid formation was observed 5 and 24 hours later. Naïve group was used as a control to verify that the adenoviral vector-treated group was not carried out in special conditions such as apoptosis or activation, and hence had the conventional same result compared to that of LacZ group.

As shown in FIG. 14, the spheroid formation was rapidly progressed with showing an improved inducing efficiency when E-cadherin was overexpressed in the mesenchymal stem cells (E-cad), unlike the results from E-cadherin inhibiting test.

Therefore, it has become clear that E-cadherin plays a key role in the spheroid formation of the mesenchymal stem cells.

### (2) Effects on ERK and/or AKT

The phosphorylation by extracellular signal-regulated kinase (ERK) and V-akt murine thymoma viral oncogene homolog (AKT) is the key factors in activating cells in the field of physiological mechanism of cells. Thus, the activities of ERK and AKT were tested.

The procedures of step (2) in Example 1 were repeated to induce spheroid formation, and the samples were taken at 30 minutes, 1 hour, and 3 hours after the induction. The phosphorylation of ERK and AKT is usually completed within 3 hours after various treatments, and hence the phosphorylation of ERK and AKT was examined by taking samples at the above specified time intervals.

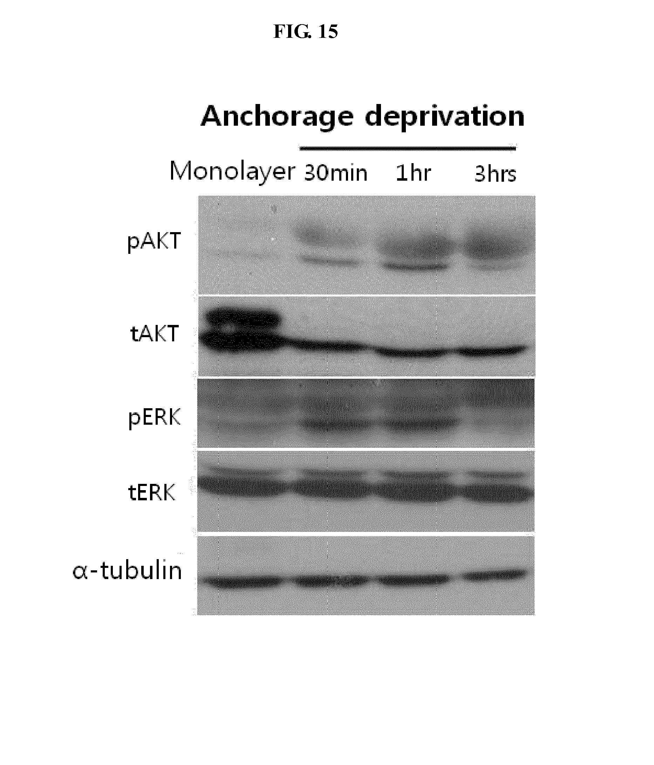

As shown in FIG. 15, as the spherical cell aggregates was formed by anchorage deprivation, the activated AKT(pAKT) and ERK(pERK) levels from the total AKT(tAKT) and ERK(tERK) levels were increased. From the above result, it can be concluded that the spherical cell aggregates formed by anchorage deprivation of the mesenchymal stem cell lead to activate both AKT and ERK, which also implies that AKT and ERK activation pathway may possibly become a consequential activation pathway by the spheroid formation of the human mesenchymal stem cell.

Next, the effects of E-cadherin on such active factors were investigated. Specifically, the single cells were treated with antibody having E-cadherin neutralization function (clone DECMA-1, sigma), and then induced to form spheroid formation, followed by subjecting to Western blot analysis. In Western blot analysis, the cells were dissolved in a reducing agent [Lysis PreMix (4°C stock) + NaF (10 M, x100) + orthovanadate (200 mM, x200) + protease inhibitor cocktail (1 tablet/10 mL)] and subjected to SDS-polyacrylamide gel electrophoresis. Then, the cells were placed on PVDF transfer membrane (Millipore) for primary antigen-antibody reaction and secondary antigen-antibody reaction using anti-rabbit IgG and anti-mouse IgG to investigate protein expression.

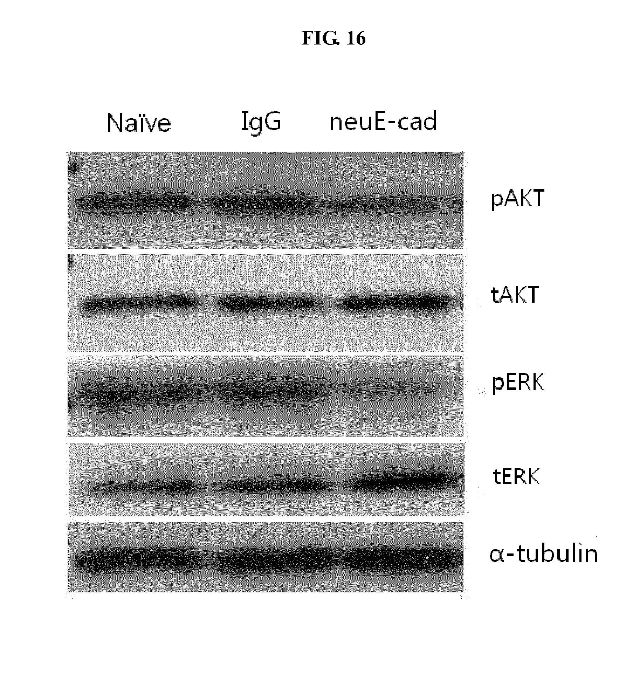

Specifically, the antibody that recognizes the cell membrane sites of E-cadherin and attaches itself thereto was used to suppress intercellular adhesion function of E-cadherin, and samples were taken to investigate the phosphorylation of ERK and AKT. The results are shown in FIG. 16. Naïve group was used as a control to verify that the antibody-treated group was not carried out in a special condition such as apoptosis or activation, and hence had the same result as compare to that of IgG group.

As shown in FIG. 16, there was no significant change in pAKT and pERK levels in IgG-treated group (control) and Naïve group, while, a reduction in AKT and ERK activation, *i.e*. pAKT and pERK levels, was detected in E-cadherin function inhibited group (neu E-cad).

Additionally, the above result was reconfirmed by using E-cadherin overexpressing adenoviral vector (E-cadherin adenoviral vector). In the same manner of the conventional viral vector transduction, a viral supernatant was added to the mesenchymal stem cell having 70% confluence by adherent culture to induce the expression of E-cadherin. After 24 hours for transduction, the cells were allowed to stabilize for 24 hours and separated to single cells. The separated single cells were induced to the spheroid formation in bFGF-free ESM by employing the low attachment dish and the samples were collected.

A control group (LacZ group) employs the same vector as the adenoviral vector of E-cadherin group except comprising LacZ gene instead of E-cadherin. Adenoviral vector-untreated group (Naïve group) was used as a control group for adenoviral vector-treated groups (E-cad and LacZ groups). Naïve group was used as a control to verify that the adenoviral vector-treated group was not carried out in special conditions such as cell death or activation, and hence conventional shows that same result compared to that of LacZ group.

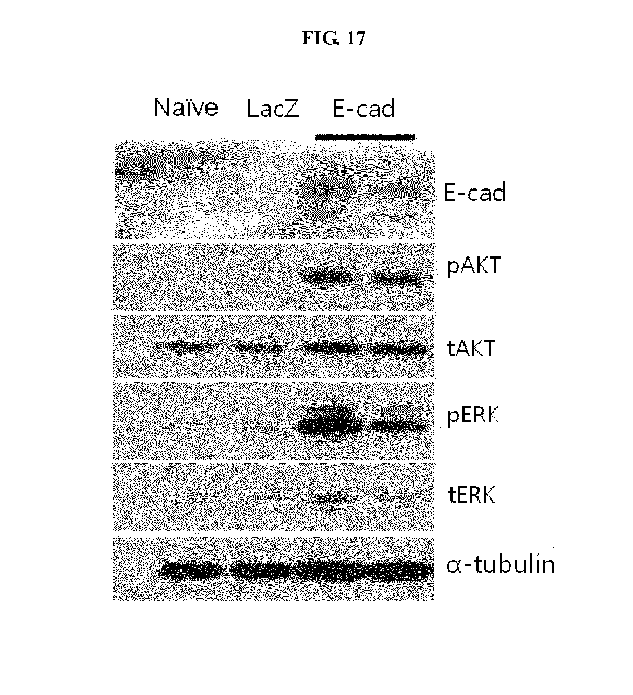

As shown in FIG. 17, it was observed that ERK and AKT was noticeably activated in E-cadherin overexpressing group (E-cad group) as compared to naïve and LacZ groups, by demonstrating increased levels of pAKT and pERK.

### (3) Effects on cell growth and death

The effects of E-cadherin on cell growth and death of mesenchymal stem cells were investigated.

Specifically, the cell growth in E-cadherin overexpressing group (E-cad group), Naive group and LacZ group were examined using flow cytometry analysis. The mesenchymal stem cells prepared in the same manner as disclosed in step (1) of Example 1 were treated with E-cadherin overexpressing adenoviral vector according to the method disclosed in step (1) of Example 4, and cultured for 24 hours to induce the spheroid formation. The spheroids thus obtained were separated to single cells, and nuclei of the single cells were stained. Subsequently, the stained cells were subjected to flow cytometry analysis for analyzing cell cycle. The cell growth (%) in each group was evaluated at S phase (synthetic phase) of the cell cycle in which the cell growth is active.

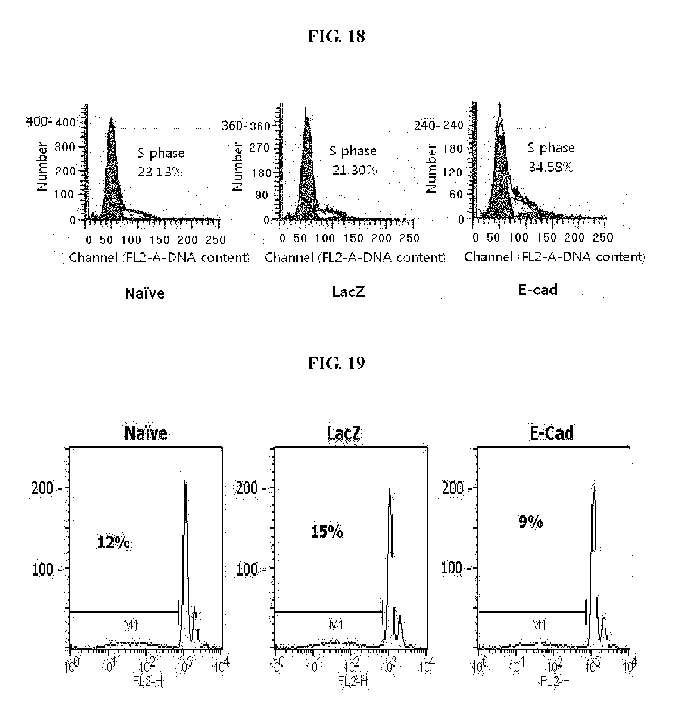

As shown in FIG. 18, E-cadherin overexpressing group (E-cad) showed the increased cell growth in S phase which is an important growth period of the mesenchymal stem cell, as compared to Naïve and LacZ groups.

In addition, as shown in FIG. 19, when E-cadherin is overexpressed the percentage of M1 phase in which apoptosis is developed was decreased and it demonstrates the cell death is reduced.

### (4) Effects on VEGF secretion

The effects of E-cadherin on vascular endothelial growth factor (VEGF) secretion of the mesenchymal stem cells were investigated. VEGF is a key factor in treating the ischemic heart disease.

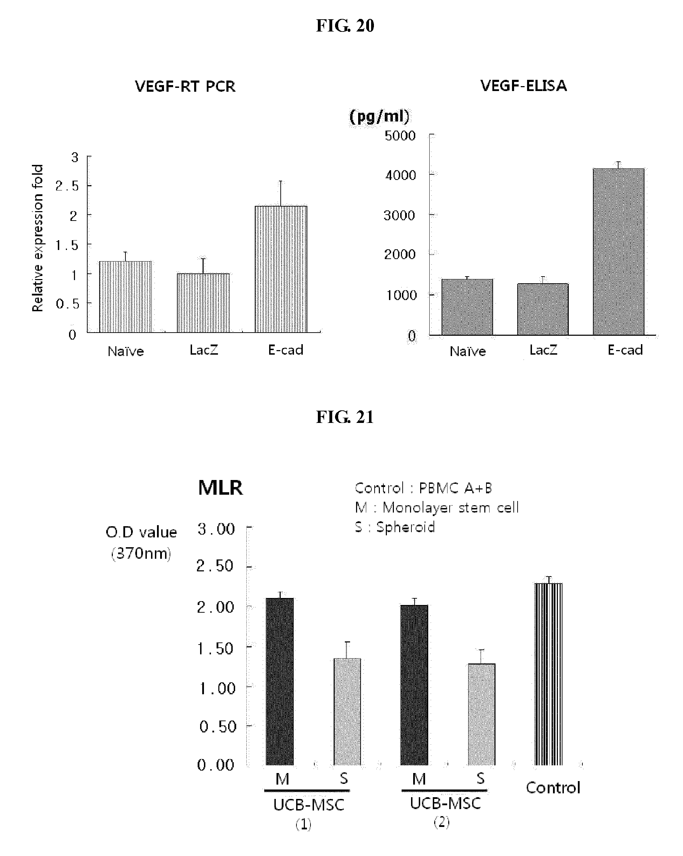

Specifically, real-time PCR and ELISA analysis using antigen-antibody reaction were conducted on E-cadherin overexpressing group, Naive group and LacZ group, and each of their mRNA and protein expression levels was compared and shown in FIG. 20.

The stem cells were treated with the adenoviral vector and cultured. After 48 hours, RNA was extracted to synthesize cDNA, followed by a quantitative analysis of RNA levels using VEGF-specific primer set (custom-made, Bioneer, Korea) (VEGF-real-time PCR). Also, the stem cells were treated with the adenoviral vector and cultured. After 48 hours, the culture solution thus obtained was subjected to VEGF antigen-antibody reaction, followed by a quantitative analysis of VEGF in protein expression levels (VEGF-ELISA).

As shown in FIG. 20, E-cadherin overexpressing group showed relatively increased VEGF levels in both mRNA and protein expression.

From the above result, it can be concluded that E-cadherin does not only act as an inducing factor for spheroid formation of human mesenchymal stem cell, but also acts as a modulator of various *in vivo* activities. In summary, it is clear that E-cadherin promotes the spheroid formation of human mesenchymal stem cell, and induces the high activity of the spherical cell aggregates.

### Example 5: Analysis of immunomodulatory activity of UCB-MSC by aggregate formation

### (1) MLR (Mixed Lymphocyte Reaction)

In order to evaluate immunomodulatory activities after the aggregate formation from umbilical cord blood-derived mesenchymal stem cells (UCB-MSCs), a mixed lymphocyte reaction (MLR) test was performed in a test tube using two UCB-MSC samples from different donors.

Specifically, allogenic human peripheral blood cells obtained from two different donors were co-cultured to induce alloimmune response. The cell growth of each sample was inhibited, and the resulting cells were co-cultured with UCB-MSCs cultured by a plate-adhesive culture (monolayer stem cells) or UCB-MSCs in the form of aggregates (stem cell aggregates), and the values of MLR test were evaluated for comparing the immunomodulatory activities between the stem cells.

In the experiment, the UCB-MSCs were used after monolayer-culturing UCB-MSCs in MEM-α medium supplemented with 10% FBS in a ratio of 5 x 10⁵/cm² to have 80-90% confluency in 175T culture dish. The resulting UCB-MSCs were treated with mitomycin C (10 µg/mL) for 1 hour under anchorage deprivation states, and then used to apply to the plate-adhesive culture and aggregate formation culture, respectively.

In order to form the stem cell aggregates, the UCB-MSCs treated with mitomycin C were cultured by hanging drop method on a lid of culture media dish in DMEM/F12 medium (containing 20% Knock-out SR, 0.1mM β-mercaptoethanol, 1% non-essential amino acid, 50 IU/mL penicillin and 50 µg/mL streptomycin) in a concentration of 2 x 10³ cells /20 µl for 24 hours.

Monolayer stem cells (2 x 10⁴ cells) and stem cell aggregates (2 x 10³ cells) were transferred to each well in a 96-well plate as negative controls, respectively. For a positive control, 2 x 10⁵ cells of the human peripheral blood cells obtained from two different donors were co-cultured to induce alloimmune response. For a test group, 2 x 10⁵ cells of the human peripheral blood cells obtained from two different donors were transferred to each well containing 2 x 10⁴ cells of the monolayer stem cells and 2 x 10³ cells of the stem cell aggregates, respectively, and co-cultured to investigate the inhibitory activities on the alloimmune response. After 5 days of the cultivation, the cell growth and spheroid formation were observed with a microscope. Next, the samples were treated with BrdU 5 days after the cultivation, and DNA of the newly synthesized cells within 24 hours was observed.

As a result shown in FIG. 21, the stem cell aggregates (S) inhibited the alloimmune response at least 37% more than the monolayer stem cells (M), which demonstrates the stem cell aggregates have superior immunosuppressing activities.

### (2) Prostaglandin E₂ (PGE₂) secretion

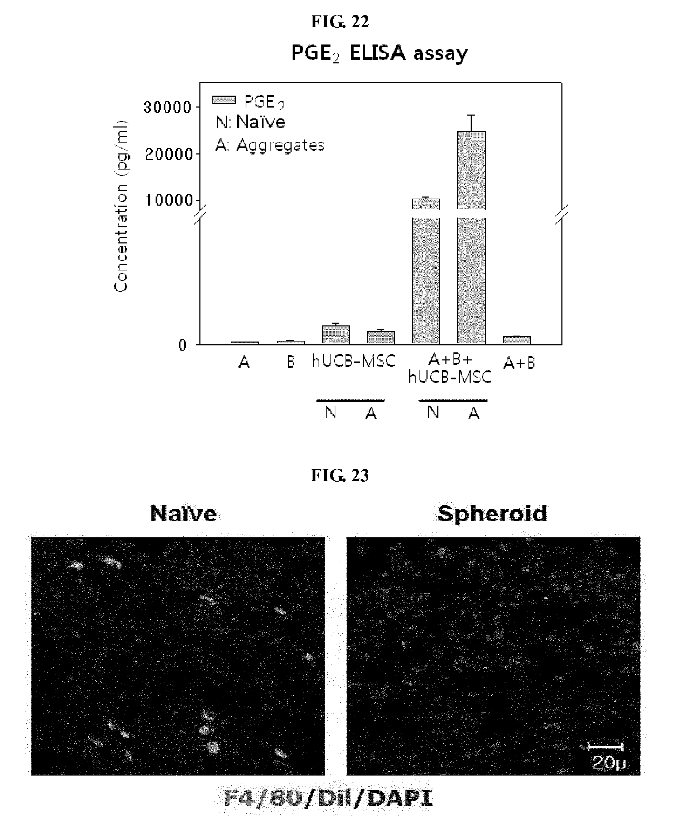

Secretion levels of PGE₂, which is a known immune modulator, were measured by ELISA (Cayman Chemical Company, prostaglandin E₂ ELISA Kit (catalog No. 514010)) from the MLR culture medium obtained in (1). The culture medium was allowed to react with a capture antibody at 4°C for 18 hours, subjected to a color reaction at a room temperature for 90 minutes, and then analyzed. As a result of ELISA analysis, FIG. 22 shows that the secretion levels of PGE₂ significantly increased after the aggregate formation under the alloimmune response-induced condition (N: monolayer stem cells, and A: stem cell aggregates). The results indicate that immunomodulation function of the UCB-MSCs was enhanced by the aggregate formation compared than that of the UCB-MSCs obtained by the plate-adhesive culture.

### (3) Immunogenicity

To investigate the effects of the aggregate formation on immunogenicity of UCB-MSCs, a tissue analysis was performed. Specifically, UCB-MSCs cultured by a plate-adhesive culture and UCB-MSCs in the form of aggregates were injected respectively to myocardium of rat models with ischemic heart disease. The heart tissue samples were collected and stained with immunocyte marker F4/80 to analyze the infiltration of immunocytes around the ischemic tissues, and a green marker was used as secondary antibodies. At this time, the injected cells were stained with DiI before the injection for easier traceability.

As shown in FIG. 23, the tissue injected with the UCB-MSCs in the form of aggregates (Spheroid) showed a significantly less number of immunocytes as compared to that of the UCB-MSCs cultured by the plate-adhesive culture (Naïve). The result indicates that aggregate formation lowered the immunogenicity of UCB-MSCs.

### Example 7: Enhancement of the effects of UCB-MSCs on chondrocyte death and chondrogenesis by the aggregate formation

### (1) Chondrocyte death inhibiting effects

It is well known that UCB-MSCs are capable of differentiating into chondrocytes, inhibit cell death caused by various secretion factors and have an anti-inflammatory effect, and thus it has been attempted to apply them for the treatment of cartilage injury diseases. Accordingly, it was verified whether or not the aggregate formation of UCB-MSCs attributes to the enhancement or improvement in the effects of UCB-MSCs on the chondrogenesis and the inhibition of the chondrocyte death caused by cartilage injury and arthritis.

Rabbit chondrocytes were monolayer-cultured in 3 mL of DMEM medium (10% FBS and 50 µg/mL Gentamicin) by employing a 10 cm² culture dish in a ratio of 5 x 10⁴ cells/cm². One day before co-culturing, the plate-adhesive cultured UCB-MSCs (naïve hUCB-MSC) were cultured in a concentration of 5 x 10⁵ cells/3 mL on the upper side of a trans-well. The aggregates of the UCB-MSCs (spheroid hUCB-MSC) was prepared by culturing UCB-MSCs in DMEM/F12 medium (20% Knock out SR, 0.1mM β-mercaptoethanol, 1% non-essential amino acid, 50 IU/mL penicillin and 50 µg/ml streptomycin) on a lid of culture dish (1 x 10⁴ cell/20 µL) by hanging drop method, one day before co-culturing. Six days after the cultivation of rabbit chondrocytes, co-culturing of separated cells was performed using a trans-well. Co-culturing was carried out by placing the trans-well containing cultured Naïve hUCB-MSC on the rabbit chondrocyte culture dish. In case of spheroid hUCB-MSC, 50 spheroids formed as above were transferred to 3 mL of DMEM medium (10% FBS and 50 µg/mL Gentamicin), and placed on a trans-well, and co-cultured with the rabbit chondrocytes. At this time, the medium was added with 500 µM of sodium nitroprusside for inducing chondrocyte death.

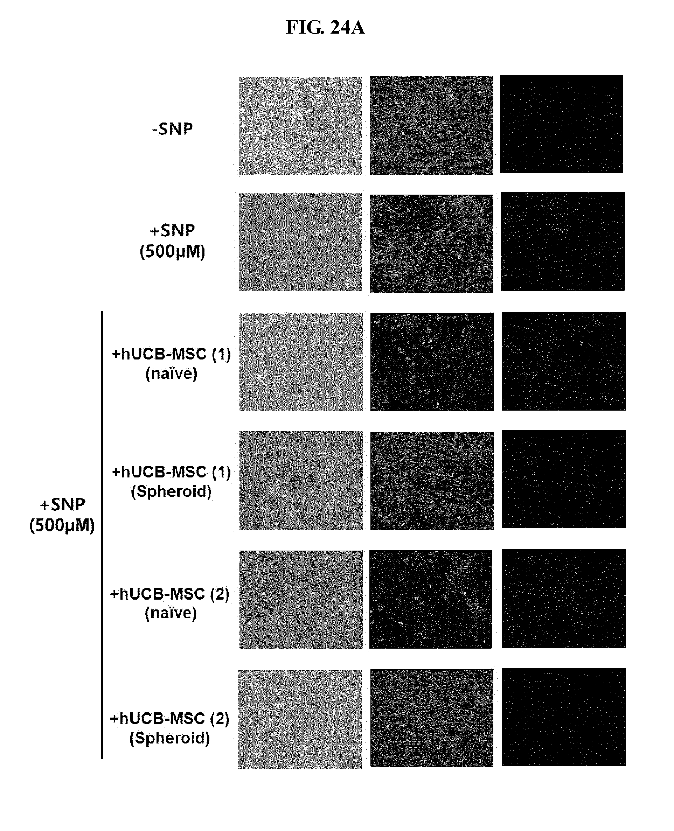

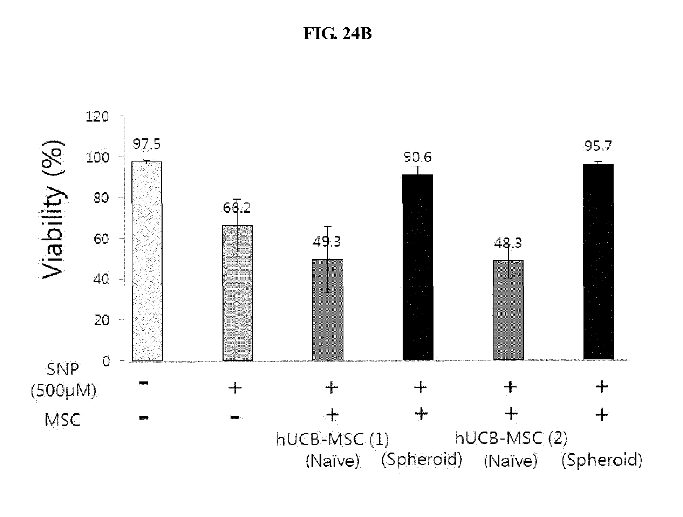

As shown in FIG. 24A, the live/dead stain results demonstrate that the chondrocyte death is noticeably decreased, and in FIR. 24B, the degree of inhibition on the chondrocyte death in hUCB-MSC (1) and hUCB-MSC (2) were 90.6 ± 4.4%, and 95.7 ± 1.2%, respectively, which significantly increased due to the spheroid formation as compared to the control group (66.2 ± 13.0%).

### (2) Effects on chondrogenesis

In 10 week-old New Zealand white rabbits, lateral skin of knee joint, subcutaneous tissue and knee capsule were incised to expose knee joint. A defect in articular cartilage of the center of trochlear groove was created using a biopsy punch having a 5 mm diameter, to prepare an articular cartilage defect model, and followed by hemostasis using sterilized gauze for 20 seconds on the defect site. Subsequently, each 5 x 10⁶ cells of two cell groups (High cell and Low cell groups) classified based on chondrogenic differentiation and regenerative capabilities of UCB-MSCs; a control group (normal human lung fibroblast cells); and an aggregate group prepared from Low cells were mixed with 4% hyaluronic acid, and injected to the defective site. The defective site (knee capsule, subcutaneous tissue and lateral skin of knee joint) was sutured, and the rabbits were bred for 10 weeks. Thereafter, enhancement of chondrogenic capability of UCB-MSCs induced by the aggregation formation was analyzed.

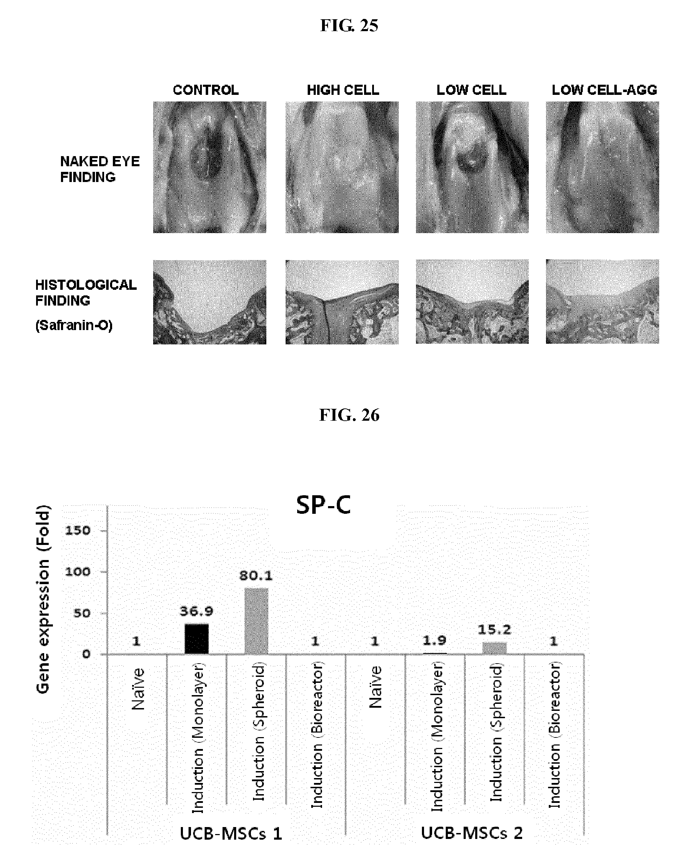

As can be seen in FIG. 25, assessment of the degrees of the cartilage injury, which was obtained by macroscopic and histologic (tissue staining) analyses 10 weeks after the injection, was performed in accordance with the methods of Pineda et al. (1992, Acta Anat (Basel)) and Wakitani et al. (1994, J Bone Joint Surg Am). Wherein, a lower grade of the defective cartilage indicates higher recovery by chondrogenesis. As an outcome, High cell group (5.00 ± 2.24) and aggregated Low cell group (6.00 ± 1.22) resulted higher values than Low cell group (7.40 ± 1.52) and control group (7.20 ± 1.48), as expected. The outcome represents that the aggregate formation inhibits the chondrocyte death and enhances chondrogenesis.

### Example 8: Improved lung regenerating effects by the aggregate formation of UCB-MSC in lung injury model

### (1) Expression of VEGF related to the activity of stem cells regenerating pneumocyte

It has been reported that the effects of UCB-MSC such as inhibitory effects on cell death and anti-inflammatory activity are caused by various secretion factors. In particular, it is known that VEGF is related with pulmonary capillary regeneration, proliferation of pneumocytes and inhibition of pneumocyte death for pulmonary recovery in pulmonary diseases [Varet J. et al., Am J physiol Lung Cell Mol Physiol, 298, L768-L774 (2010); and Kuhn H et al., Respirology 15, 343-348 (2010)]. In this regard, it is expected that the aggregate formation of UCB-MSCs increases the VEGF secretion levels, thereby facilitating the lung regeneration in lung injury model. Therefore, the VEGF secretion levels of UCB-MSCs before and after the aggregate formation were analyzed by ELISA using an anti-VEGF antibody (R&D systems, ELISA kit cat #DY293B).

In the result, VEGF secretion levels of UCB-MSCs after aggregate formation exhibited a three-fold increase as compared to that of UCB-MSCs before aggregate formation. It means that the aggregate formation increases the secretion levels of VEGF, which is a major therapeutic agent for pulmonary disease, thereby improving the pulmonary recovery.

### (2) Enhanced pneumocyte differentiation by the aggregate formation of UCB-MSC

It has been known that UCB-MSCs are differentiated into pneumocytes, and have capabilities of the pneumocyte formation or regeneration by attached into pulmonary tissues, inhibition of lung fibrosis and anti-inflammatory activity, and thus, attempts were made to develop a therapeutic agent using same for treatment of pulmonary diseases. Particularly, SP-C (surfactant protein C) released from pneumocytes for regenerating pneumocytes is an important factor in the treatment of pulmonary disease. Therefore, it is investigated whether the therapeutic effect of UCB-MSC can be improved or enhanced by the aggregate formation.

In order to verify the effect of UCB-MSCs on pneumocyte differentiation after the aggregate formation, SP-C gene expression was evaluated *in vitro* using UCB-MSCs obtained from two different donors. In the experiment, the UCB-MSCs were used after monolayer-culturing UCB-MSCs in MEM-α medium supplemented with 10% FBS on 175T culture dish at a ratio of 5 x 10³ cells/cm² to 50-60% confluency. For monolayer culture, UCB-MSCs thus obtained were grown on a 75T culture dish.

The aggregate formation was carried out by using two different methods of hanging drop and bioreactor. In the hanging drop method, the UCB-MSCs were cultured in DMEM/F12 medium (containing 20% Knock out SR, 0.1mM β-mercaptoethanol, 1% non-essential amino acid, 50 IU/mL penicillin and 50 mg/mL streptomycin) by employing a lid of culture dish in a concentration of 2 x 10³ cells/20 µl for 24 hours. Proliferation of the cells and formation of spheroids were checked under microscope, and the spheroids were transferred to a 35π culture dish for further cultivation. In the bioreactor method, the UCB-MSCs were cultured in a spinner flask in a concentration of 5 x 10⁵ cells /mL at a constant rate of 70 rpm.

The monolayer culture group and the two aggregate groups were cultured for 5 days, and subjected to RNA extraction, followed by cDNA synthesis. Next, SP-C expression level was analyzed using Real time PCR.

As shown in FIG. 26, the level of SP-C expression from the UCB-MSC aggregates prepared by the hanging drop method exhibits a noticeable increase as compared to those from monolayer culture and the aggregates prepared by the bioreactor method, which indicates that therapeutic effectiveness of the aggregates prepared by the hanging drop method is superior to the aggregates prepared by the bioreactor method. In the result, the expression of pneumocyte differentiation factor, SP-C, from the aggregates prepared by the hanging drop method exhibited 15 to 80-fold increase, as compared to that of the bioreactor group, and 2 to 8-fold increase, as compared to that of Monolayer group. It means that the aggregate formation by the hanging drop method increases the expression of SP-C, which is a major therapeutic agent for pulmonary disease, thereby improving the pulmonary recovery.

As shown in the results of the above Examples, it is expected that the aggregate formation of UCB-MSCs increases the levels of various functional secreted factors and decreases immunogenicity of the cells, thereby enhancing the therapeutic effect of the cells as a cell therapy agent in the treatment of chondropathy and pulmonary diseases, as well as inflammatory diseases.

### Example 9: Method for forming spheroids of UCB-MSC using a rocker

In order to form MSC aggregates, MSCs were induced to form aggregates by rocking them using a rocker. MSC cells in 10,000 ~ 20,000 cells/cm² were cultured in α-DMEM medium supplemented with 10% FBS. A compact rocker CR95 (FinePCR CO., LTD.) was placed in a CO₂ cultivator at 37°C, and the MSC cells were cultured for 24 hours at a rocking speed of 8~12 rpm. To prevent the MSC attachment on the surface of the dish, a non-treated bacterial culture dish was used.

As a result, the aggregate formation of MSC decreased as the rocking speed increased. Also, it was observed that the range of the aggregate size varied depending on the rocking speed, and the aggregate size varied although the rocking speed remained unchanged.

While the invention has been described with respect to the above specific embodiments, it should be recognized that various modifications and changes may be made to the invention by those skilled in the art which also fall within the scope of the invention as defined by the appended claims.

## Claims

1. A method for preparing a highly active human mesenchymal stem cell aggregate, comprising culturing a human mesenchymal stem cell against gravity to form a spherical cell aggregate.

2. The method of claim 1, wherein said human mesenchymal stem cell is cultured in a culture drop positioned against gravity.

3. The method of claim 1, wherein said human mesenchymal stem cell is cultured in a serum-free medium comprising a serum replacement (SR).

4. The method of claim 3, wherein said serum-free medium is a human embryonic stem cell culture medium that does not contain basic fibroblast growth factor (bFGF).

5. The method of claim 1, wherein the mesenchymal stem cell is originated from human umbilical cord-blood.

6. A method for preparing a highly active human mesenchymal stem cell aggregate, comprising culturing a human mesenchymal stem cell to form a spherical cell aggregate, wherein the amount of E-cadherin in the mesenchymal stem cell is increased during the culture.

7. The method claim 6, wherein the amount of E-cadherin is increased by introducing an expression vector of E-cadherin into the mesenchymal stem cell.

8. A highly active human mesenchymal stem cell aggregate prepared by the method of any one of claims 1 to 7.

9. A cell therapeutic agent comprising the highly active human mesenchymal stem cell aggregate of claim 8.

10. The cell therapeutic agent of claim 9, which is used for generating adipocytes, osteocytes, chondrocytes, myocytes, neurocytes, cardiomyocytes, hepatocytes, islet beta cells, angiocytes, or pneumocytes.

11. The cell therapeutic agent of claim 9, which is used for any one selected from the group consisting of: treatment of a pulmonary disease; suppression or treatment of an inflammation caused by a pulmonary disease; regeneration of a pulmonary tissue; and suppression of fibrosis in a pulmonary tissue.

12. The cell therapeutic agent of claim 9, which is used for the treatment of a cardiovascular disorder.

13. The cell therapeutic agent of claim 9, which is used for angiogenic therapy.

14. The cell therapeutic agent of claim 9, which is used for the enhancement of immunomodulatory activity.

15. The cell therapeutic agent of claim 9, wherein said cell therapeutic agent reduces any one selected from immunifacient activity, penetration of immunocytes and immunogenicity.

16. The cell therapeutic agent of claim 9, which is used for chondrogenesis.

17. The cell therapeutic agent of claim 9, which is used for suppressing an inflammatory reaction.

18. A cell therapeutic method comprising administering the highly active human mesenchymal stem cell aggregate of claim 8 to a subject in need thereof.

19. The cell therapeutic method of claim 17, which is used for generating adipocytes, osteocytes, chondrocytes, myocytes, neurocytes, cardiomyocytes, hepatocytes, islet beta cells, angiocytes, or pneumocytes; treatment of a pulmonary disease; suppression or treatment of an inflammation caused by a pulmonary disease; regeneration of a pulmonary tissue; and suppression of fibrosis in a pulmonary tissue; treatment of a cardiovascular disorder; enhancement of immunomodulatory activity; reduction of immunifacient activity, penetration of immunocytes, or immunogenicity; chondrogenesis; or suppression of an inflammatory reaction.
